# EUROPEAN PATENT SPECIFICATION

(11) **EP 2 788 511 B1**
(45) Date of publication and mention of the grant of the patent: **27.09.2017**
(21) Application number: 12813446.7
(22) Date of filing: 06.12.2012
(51) Int. Cl.: C12R 1/225, C12P 19/42, A61K 35/747, C12N 1/20, A23L 33/135, A23L 33/15

(54) **VITAMIN B12 PRODUCING LACTOBACILLUS REUTERI STRAINS**
VITAMIN B12-PRODUZIERENDE LACTOBACILLUS REUTERI-STÄMME
SOUCHES DE LACTOBACILLUS REUTERI PRODUISANT DE LA VITAMINE B12

(30) Priority: 09.12.2011 IT MI20112238
(43) Date of publication of application: 15.10.2014
(73) Proprietor: Probiotical S.p.A., 28100 Novara (IT)
(72) Inventor: MOGNA, Giovanni, I-28100 Novara (IT); STROZZI, Gian Paolo, I-28100 Novara (IT); MOGNA, Luca, I-28100 Novara (IT)
(74) Representative: Hoffmann Eitle
(86) International application number: PCT/IB2012/002612
(87) International publication number: WO 2013/084052

(56) References cited:
- EP-A1- 1 602 716
- WO-A1-2006/013588
- WO-A1-2009/138300
- WO-A1-2010/063601
- WO-A1-2012/143787
- HUGENSCHMIDT S ET AL: "Screening of a natural biodiversity of lactic and propionic acid bacteria for folate and vitamin B12 production in supplemented whey permeate", INTERNATIONAL DAIRY JOURNAL, ELSEVIER APPLIED SCIENCE, BARKING, GB, vol. 20, no. 12, 1 December 2010 (2010-12-01), pages 852-857, XP027282299, ISSN: 0958-6946 [retrieved on 2010-06-12]
- MOLINA V C ET AL: "Lactobacillus reuteri CRL 1098 prevents side effects produced by a nutritional vitamin B12 deficiency", JOURNAL OF APPLIED MICROBIOLOGY, OXFORD, GB, vol. 106, no. 2, 1 February 2009 (2009-02-01), pages 467-473, XP002621372, ISSN: 1364-5072, DOI: 10.1111/J.1365-2672.2008.04014.X [retrieved on 2009-01-07]
- SIUTA-CRUCE P ET AL: "IMPROVING PROBIOTIC SURVIVAL RATES MICROENCAPSULATION PRESERVES THE POTENCY OF PROBIOTIC MICROORGANISMS IN FOOD SYSTEMS", FOOD TECHNOLOGY, INSTITUTE OF FOOD TECHNOLOGISTS, CHICAGO, IL, US, vol. 55, no. 10, 1 October 2001 (2001-10-01), pages 36,38-40,42, XP001108329, ISSN: 0015-6639
- F. SANTOS ET AL: "Effect of Amino Acid Availability on Vitamin B12 Production in Lactobacillus reuteri", APPLIED AND ENVIRONMENTAL MICROBIOLOGY, vol. 75, no. 12, 17 April 2009 (2009-04-17), pages 3930-3936, XP055030882, ISSN: 0099-2240, DOI: 10.1128/AEM.02487-08
- FILIPE SANTOS ET AL: "High-Level Folate Production in Fermented Foods by the B12 Producer Lactobacillus reuteri JCM1112", APPLIED AND ENVIRONMENTAL MICROBIOLOGY, AMERICAN SOCIETY FOR MICROBIOLOGY, US, vol. 74, no. 10, 1 May 2008 (2008-05-01), pages 3291-3294, XP002621373, ISSN: 0099-2240, DOI: 10.1128/AEM.02719-07 [retrieved on 2008-03-14]
- J.G. LEBLANC ET AL: "B-Group vitamin production by lactic acid bacteria - current knowledge and potential applications", JOURNAL OF APPLIED MICROBIOLOGY, vol. 111, no. 6, 1 December 2011 (2011-12-01), pages 1297-1309, XP055030879, ISSN: 1364-5072, DOI: 10.1111/j.1365-2672.2011.05157.x

## Description

The present invention relates to a composition comprising at least two selected probiotic bacterial strains belonging to the species *Lactobacillus reuteri* as a vitamin B12 producer. Finally, the present invention relates to a food composition or supplement or pharmaceutical composition comprising a selected bacterial strain belonging to the species *Lactobacillus reuteri* as a vitamin B12 producer.

It is well known that Cobalamin (vitamin B12) plays an important role in the production of red blood cells and is essential for proper functioning of the nervous system.

Furthermore, it is well known that four chemical forms can make reference to Cobalamin (vitamin B12) depending on the type of chemical group (R) which binds to the cobalt ion in its structural formula. The group (R) can be:
- hydrocyanic, -CN (cyanocobalamin)
- hydroxyl, -OH (hydroxocobalamin)
- methyl, -CH₃ (methylcobalamin)
- 5-deoxyadenosyl(5'-deoxyadenosylcobalamin).

The metabolically active forms are methyl- and 5'-deoxyadenosylcobalamin.

Hydroxocobalamin is the natural form in which vitamin B12 is usually taken in through the diet.

However, for food and pharmaceutical use of said vitamin, reliance must be made on the most stable form, represented by cyanocobalamin, which is chemically synthesized. Cyanocobalamin does not exist in nature except in rare cases.

Therefore, the cyanocobalamin produced synthetically is an artificial substance which is formed during industrial extraction processes, as use is made of papaya, a protease which is activated by the addition of CN-. In practice, during industrial synthetic processes, a transformation of hydroxocobalamin into cyanocobalamin is effected, because the latter form of vitamin B12 is more stable when exposed to air and more easily crystallisable, thus favouring production yields. For this reason, the majority of food products, food supplements and pharmaceutical products contain cyanocobalamin.

Therefore, the possibility of producing vitamin B12 naturally in the form of hydroxocobalamin as an alternative to vitamin B12 produced synthetically in the form of cyanocobalamin is viewed with much interest and responds to a strongly felt need of industry operators.

Hugenschmidt, S et al (International Dairy Journal 2010, vol 20, no 12, pages 852-857) discloses the intracellular vitaim B12 production of three *L. reuteri* strains after 24 hours of incubation in supplemented whey permeate medium, with yields in a range from 0.058 to 0.115 µg.mL⁻¹.

Molina, V. C. et al (Journal of Applied Microbiology 2009, vol 106, no 2, pages 467-473) relates to a *L reuteri* strain capable of producing pseudovitamin B12

Therefore, there remains a need to have a method for producing vitamin B12 naturally.

In particular, there remains a need to have a method for producing hydroxocobalamin naturally.

Finally, it is well known that meat contains vitamin B12.

However, for people who do not consume meat-based products, such as vegetarians, it may be difficult to procure a daily amount of vitamin B12 sufficient to meet daily requirements.

Therefore, the availability of non-meat-based food products rich in vitamin B12 would enable vegetarians to take in more easily a daily dose of vitamin B12 sufficient to meet their daily requirements.

In an ambodiment, the subject matter of the present invention is a composition comprising at least two selected probiotic bacterial strains belonging to the species *Lactobacillus reuteri,* as set forth in the appended claim.

The subject matter of the present invention further relates to a food composition or supplement or pharmaceutical composition for use as set forth in the appended claim.

The subject matter of the present invention further relates to a pharmaceutical composition for use in the preventive and/or curative treatment of subjects suffering from a pathology due to a vitamin B12 deficiency or deficit, as set forth in the appended claim.

The subject matter of the present invention further relates to a pharmaceutical composition for use in the preventive and/or curative treatment of subjects suffering from pernicious anaemia, as set forth in the appended claim.

The subject matter of the present invention further relates to a pharmaceutical composition for use in the treatment of subjects who take vitamin C, as set forth in the appended claim.

Further preferred embodiments of the present invention will be set forth and illustrated below without intending to limit the scope of the present invention in any way.

Table 1 shows the concentration values (mean value over a number of tests) of vitamin B12 (expressed in µg/litre) measured at OD₆₀₀.

After intense research activity the Applicant selected, among the probiotic bacterial strains selected from the group comprising the bacterial strains belonging to the species *Lactobacillus reuteri,* only those capable of producing vitamin B12.

The strain, as a vitamin B12 producer, is selected from the group consisting of:
- *Lactobacillus reuteri* DSM 23877 - (LRE01),
- *Lactobacillus reuteri* DSM 23878 - (LRE02),
- *Lactobacillus reuteri* DSM 23879 - (LRE03),
- *Lactobacillus reuteri* DSM 23880 - (LRE04).

Advantageously, the strain is *Lactobacillus reuteri* DSM 23878 (LRE02). The strain *Lactobacillus reuteri* DSM 23878 (LRE02) is in association with at least one strain selected from the group consisting of:
- *Lactobacillus reuteri* ATCC 55730,
- *Lactobacillus reuteri* DSM 17938, and
- *Lactobacillus reuteri* DSM 16143;

In one embodiment, the strain *Lactobacillus reuteri* DSM 23878 (LRE02) is in association with at least one strain selected from the group consisting of:
- *Lactobacillus reuteri* ATCC 55730,
- *Lactobacillus reuteri* DSM 17938.

The food or nutraceutical composition or supplement or pharmaceutical composition for use according to the present invention comprises at least one bacterial strain, which is the strain *Lactobacillus reuteri* DSM 23878 (LRE02).

Preferably, the food composition or supplement or pharmaceutical composition comprises two strains or three strains, selected from among the above-mentioned ones.

Advantageously, the food or nutraceutical composition or supplement or pharmaceutical composition further comprises the strains *Lactobacillus reuteri* ATCC 55730 and *Lactobacillus reuteri* DSM 17938 or, alternatively, comprises or consists of the three strains *Lactobacillus reuteri* ATCC 55730, *Lactobacillus reuteri* DSM 17938 and *Lactobacillus reuteri* DSM 16143.

The food or nutraceutical composition or supplement or pharmaceutical composition can comprise or, alternatively, consist of:
i) the strain *Lactobacillus reuteri* ATCC 55730 and *Lactobacillus reuteri* DSM 23878 (LRE02), or
ii) the strain *Lactobacillus reuteri* DSM 17938 and *Lactobacillus reuteri* DSM 23878 (LRE02), or
iii) the strain *Lactobacillus reuteri* DSM 16143 and *Lactobacillus reuteri* DSM 23878 (LRE02), or
iv) the three strains *Lactobacillus reuteri* ATCC 55730, *Lactobacillus reuteri* DSM 17938 and *Lactobacillus reuteri* DSM 23878 (LRE02), or
v) the three strains *Lactobacillus reuteri* DSM 23878 (LRE02), *Lactobacillus reuteri* DSM 17938 and *Lactobacillus reuteri* DSM 16143, or
vi) the three strains *Lactobacillus reuteri* DSM 23878 (LRE02), *Lactobacillus reuteri* ATCC 55730 and *Lactobacillus reuteri* DSM 16143.

The aforesaid bacterial species are present in an amount comprised from 0.1 to 75% by weight, preferably from 0.5 to 15% by weight; even more preferably from 1 to 10% by weight, relative to the total weight of the composition or supplement. However, said percentage relative to the total weight of the composition depends on the product type of the composition or supplement. For example, in a capsule the amount of said bacterial species is preferably greater than 40%.

In a preferred embodiment, the composition contains bacteria in a concentration comprised from 1x10⁶ to 1x10¹¹ CFU/g of mixture, preferably from 1x10⁸ to 1x10¹⁰ CFU/g of mixture.

In a preferred embodiment, the composition contains bacteria in a concentration comprised from 1x10⁶ to 1x10¹¹ CFU/dose, preferably from 1x10⁸ to 1x10¹⁰ CFU/dose.

The dose may be comprised from 0.2 to 10 g, for example it is 0.25 g, 1 g, 3 g, 5 g or 7 g.

The probiotic bacteria used in the present invention can be in solid form, in particular in powder, dehydrated powder, spray or lyophilized form.

In a preferred embodiment of the invention, the food composition or supplement or pharmaceutical composition can further comprise prebiotic fibres and carbohydrates having a bifidogenic action, such as, for example, inulin, fructo-oligosaccharides (FOS), galacto- and trans-galactooligosaccharides (GOS and TOS), gluco-oligosaccharides (GOSα), xylo-oligosaccharides (XOS), chitosan oligosaccharides (COS), soy oligosaccharides (SOS), isomalto-oligosaccharides (IMOS), resistant starch, pectin, psyllium, arabinogalactans, glucomannans, galactomannans, xylans, lactosucrose, lactulose, lactitol and various other types of gums, acacia, carob, oat or bamboo fibre, citrus fibres and, in general, fibres containing a soluble and an insoluble portion, in a variable ratio to each other.

Advantageously, said fibre is selected from the group comprising FOS, inulin and citrus fibres, preferably in a ratio by weight of from 1:3 to 3:1.

The amount of the prebiotic fibres and/or carbohydrates having a bifidogenic action, if present, is comprised from 0.5 to 75% by weight, preferably from 1% to 40% by weight and even more preferably from 2 to 20% by weight relative to the total weight of the composition. In this case one has a supplement with symbiotic activity.

In a preferred embodiment, the food composition or supplement or pharmaceutical composition can further comprise one or more physiologically acceptable additives or excipients.

In a preferred embodiment of the invention, the food composition or supplement or pharmaceutical composition can further comprise other active ingredients and/or components, such as vitamins, minerals, bioactive peptides, substances having antioxidant, hypocholesterolemizing, hypoglycemizing, antiinflammatory or sweetening activity in an amount by weight generally comprised from 0.001% to 10% by weight, preferably from 0.5% to 5% by weight, depending in any case on the type of active component and the recommended daily dose thereofrelative to the total weight of the composition.

The food composition or supplement or pharmaceutical composition of the present invention is prepared using already known techniques available to one of ordinary skill in the art who is capable of using known machinery and apparatus and the most suitable method.

In a preferred embodiment, the food composition or supplement or pharmaceutical composition can contain elements or substances with antioxidant activity as mentioned above, in an amount comprised from 0.0001% to 30% by weight relative to the weight of the final composition, depending on the concentration of substances with antioxidant activity and/or on the recommended daily amount (RDA), where defined.

Selenium can be present in the form of sodium selenate, L-selenomethionine, sodium selenite, sodium acid selenite and selenious acid, as well as in the form of selenium-enriched microorganisms, e.g. yeast, in an amount by weight comprised from 0.0005% to 0.005% relative to the weight of the final composition, in any case sufficient to contribute a quantity of selenium preferably comprised from 10 µg to 150 µg.

Advantageously, the strains deposited by the company BIOMAN S.r.l., Via Alfieri 18, 10100 Turin, Italy, have application; namely:
- *Lactobacillus buchneri* LB26BM, deposited with the DSMZ on 05/04/2004 and having the deposit number DSM 16341, and/or
- *Lactobacillus ferintoshensis* LB6BM, deposited with the DSMZ on 17/01/2004 and having the deposit number DSM 16144, and/or
- *Lactobacillus reuteri* LB2BM, deposited with the DSMZ on 17/01/2004 and having the deposit number DSM 16143, and/or
in association with the vitamin B12 producing strains of the present invention.

Said strains, in fact, are capable of accumulating inside cells large quantities of selenium, especially in organic form, if grown in the presence of a suitable source of selenium in the culture medium.

In another preferred embodiment, the food composition or supplement or pharmaceutical composition can contain glutathione. In particular, glutathione in antioxidant form relates to reduced glutathione (or GSH). In a preferred embodiment, the composition comprises glutathione in reduced form and selenium in an amount by weight comprised from 0.5% to 25%, relative to the weight of the final composition, in association with the vitamin B12 producing strains of the present invention.

Advantageously, since glutathione can be partially inactivated if taken orally, the composition can comprise the sulphur amino acid cysteine and/or N-acetylcysteine and/or mixtures thereof.

In one embodiment of the present invention, the food composition or supplement or pharmaceutical composition can contain the above-mentioned probiotic bacteria of the present invention in microencapsulated form, i.e. coated with a composition containing at least one lipid (lipid composition), preferably of vegetable origin.

Alternatively, the food composition or supplement or pharmaceutical composition can comprise the above-mentioned probiotic bacteria of the present invention as microencapsulated bacteria and non-microencapsulated bacteria.

Said lipid composition comprises at least one lipid, and said at least one lipid is of vegetable origin. Advantageously, said lipid of vegetable origin is selected from the group comprising saturated fats. Advantageously, saturated fats are used having a melting point below 75°C, preferably comprised from 45 to 65°C.

In a preferred embodiment, said saturated fats are selected from the group comprising mono- and diglycerides of saturated fatty acids, polyglycerols esterified with saturated fatty acids and free saturated fatty acids. Preferably, said saturated fats are selected from among polyglyceryl distearate, glyceryl palmitostearate or hydrogenated vegetable fats of non-lauric origin.

In a first embodiment, the above-mentioned probiotic bacteria of the present invention are mono-coated.

In practice, a single coating is produced with a same lipid. Advantageously, the single coating is based on polyglyceryl distearate (commercial name Plurol Stearique WL 1009).

The above-mentioned mono-coated probiotic bacteria of the present invention are placed in the food composition or supplement or pharmaceutical composition of the present invention.

In a second embodiment, the above-mentioned probiotic bacteria of the present invention are double-coated. In practice, a double coating is produced, in succession, with two lipids differing from each other.

Advantageously, the two lipids are selected from among a hydrogenated palm fat (Tm=60°C) and a glycerol dipalmitostearate (Tm=57-60°C), which are sprayed onto the lyophilizate in succession, i.e. a double covering is applied on the lyophilizate: the first with the hydrogenated palm fat (for example with Revel C) and the second with the glycerol dipalmitostearate (for example Precirol Ato 5) in a ratio of 3:1 to each other, advantageously 2:1, for example 2/3 by weight of the former and 1/3 by weight of the latter.

In a preferred embodiment, the pharmaceutical composition is indicated for use in the preventive and/or curative treatment of subjects suffering from a pathology or a state of malaise due to vitamin B12 deficiencies or deficits.

In another preferred embodiment, the pharmaceutical composition is indicated for use in the preventive and/or curative treatment of subjects suffering from pernicious anaemia.

Pernicious anaemia is a disease provoked by a deficiency or deficit of vitamin B12, such as, for example, cobalamin. This disease is characterized by megaloblastic anaemia and nervous system disorders.

In these cases it is also important to evaluate the concentration of vitamin B12 and folic acid, since a deficiency of the latter similarly provokes a condition of megaloblastic anaemia, without, however, affecting the nervous system.

Therefore, in the case of pernicious anaemia it is very important to improve the anaemic condition by administering a pharmaceutical composition comprising at least one vitamin B12 producing strain of the present invention selected from the group comprising: *Lactobacillus reuteri* ATCC 53609, *Lactobacillus reuteri* DSM 20016, *Lactobacillus reuteri* ATCC 55730, *Lactobacillus reuteri* DSM 17938, *Lactobacillus reuteri* DSM 16143, *Lactobacillus reuteri* NCIMB 701359, *Lactobacillus reuteri* DSM 23877 -(LRE01), *Lactobacillus reuteri* DSM 23878 (LRE02), *Lactobacillus reuteri* DSM 23879 (LRE03), and *Lactobacillus reuteri* DSM 23880 - (LRE04), in association with at least one folic acid producing bacterial strain selected from the group comprising:
- a bacterial strain belonging to the species *Bifidobacterium adolescentis,* preferably *Bifidobacterium adolescentis* deposited with the DSMZ on 21.07.2004 and having the accession number DSM 16595;
- a bacterial strain belonging to the species *Bifidobacterium catenulatum*/*pseudocatenulatum,* preferably *Bifidobacterium catenulatum*/*pseudocatenulatum* deposited with the DSMZ on 15.06.2006 and having the accession number DSM 18350;
- a bacterial strain belonging to the species *Bifidobacterium animalis subsp. lactis,* preferably *Bifidobacterium animalis subsp. lactis* deposited with the DSMZ on 15.06.2006 and having the accession number DSM 18352;
- a bacterial strain belonging to the species *Bifidobacterium breve,* preferably *Bifidobacterium breve* deposited with the DSMZ on 21.07.2004 and having the accession number DSM 16596;
- a bacterial strain belonging to the species *Bifidobacterium pseudocatenulatum,* preferably *Bifidobacterium pseudocatenulatum* deposited with the DSMZ on 21.07.2004 and having the accession number DSM 16597, *Bifidobacterium pseudocatenulatum* deposited with the DSMZ on 21.07.2004 and having the accession number DSM 16598 and *Bifidobacterium catenulatum*/*pseudocatenulatum* deposited with the DSMZ on 15.06.2006 and having the accession number DSM 18353.

The above-mentioned strains were deposited by the company Anidral Srl (now Probiotical SpA, Via Mattei 3, Novara 28100 Italy) with the DSMZ in Germany on 21.07.2004 and 15.06.2006.

The strains *Lactobacillus reuteri* DSM 23877 -(LRE01), *Lactobacillus reuteri* DSM 23878 -(LRE02), *Lactobacillus reuteri* DSM 23879 -(LRE03) and *Lactobacillus reuteri* DSM 23880 -(LRE04) were deposited by the company Probiotical SpA (Via Mattei 3, Novara 28100 Italy) with the DSMZ in Germany on 05.08.2010.

In another preferred embodiment, the pharmaceutical composition is indicated for use in the treatment of subjects who take vitamin C, since the intake of large amounts of vitamin C (greater than 1 g) can generate cobalamin deficiencies over time. This is due to the fact that vitamin C, in the presence of iron, can act as an oxidant and form free radicals which damage cobalamin and intrinsic factor.

Therefore, a food composition or supplement or pharmaceutical composition of the present invention has valid application for those persons who take doses of vitamin C daily.

Hence the use of probiotic bacterial strains which are producers of vitamin B12 (in the form of hydroxocobalamin, which is the natural form taken in most through the diet) is an advantageous alternative compared to the use of foods or supplements or drugs containing vitamin B12 in the form of cyanocobalamin. All of the above-mentioned compositions or supplements of the present invention can be formulated in solid, lyophilized or dried form, for example in powder or granular form.

Moreover, one pharmaceutical form of interest is tablets or hard or soft capsules.

Insofar as tablets are concerned, they can comprise an inner part comprising the bacterial strains and an outer coating part. The coating can comprise water-soluble polymers and/or polymers capable of resisting the pH variations in the stomach and enabling passage into the intestinal tract.

In a preferred embodiment, the probiotic bacterial strain is selected from the group comprising the bacterial strains belonging to the species *Lactobacillus reuteri,* as a vitamin B12 producer.

In another preferred embodiment, the vitamin B12 producing strain is selected from the group comprising: *Lactobacillus reuteri* ATCC 55730, *Lactobacillus reuteri* DSM 17938 and *Lactobacillus reuteri* DSM 16143.

In another preferred embodiment, a food composition or supplement or pharmaceutical composition comprises at least one bacterial vitamin B12 producing strain, and preferably at least one additional bacterial strain capable of accumulating selenium inside the bacterial cells is further present; preferably said bacterial strain is selected from the group comprising: *Lactobacillus buchneri* LB26BM, deposited with the DSMZ on 05/04/2004 and having the deposit number DSM 16341, *Lactobacillus ferintoshensis* LB6BM, deposited with the DSMZ on 17/01/2004 and having the deposit number DSM 16144, and *Lactobacillus reuteri* LB2BM, deposited with the DSMZ on 17/01/2004 and having the deposit number DSM 16143.

In another preferred embodiment, said composition further comprises glutathione in reduced form.

In another embodiment said composition further comprises an additional bacterial strain capable of producing folic acid; preferably said strain is selected from the group comprising: a bacterial strain belonging to the species *Bifidobacterium adolescentis,* preferably *Bifidobacterium adolescentis* deposited with the DSMZ on 21.07.2004 and having the accession number DSM 16595; a bacterial strain belonging to the species *Bifidobacterium catenulatum*/*pseudocatenulatum,* preferably *Bifidobacterium catenulatum*/*pseudocatenulatum* deposited with the DSMZ on 15.06.2006 and having the accession number DSM 18350; a bacterial strain belonging to the species *Bifidobacterium animalis subsp. lactis,* preferably *Bifidobacterium animalis subsp. lactis* deposited with the DSMZ on 15.06.2006 and having the accession number DSM 18352; a bacterial strain belonging to the species *Bifidobacterium breve,* preferably *Bifidobacterium breve* deposited with the DSMZ on 21.07.2004 and having the accession number DSM 16596; a bacterial strain belonging to the species *Bifidobacterium pseudocatenulatum,* preferably *Bifidobacterium pseudocatenulatum* deposited with the DSMZ on 21.07.2004 and having the accession number DSM 16597, *Bifidobacterium pseudocatenulatum* deposited with the DSMZ on 21.07.2004 and having the accession number DSM 16598 and *Bifidobacterium catenulatum*/*pseudocatenulatum* deposited with the DSMZ on 15.06.2006 and having the accession number DSM 18353.

In another preferred embodiment, said pharmaceutical composition is indicated for use in the preventive and/or curative treatment of subjects suffering from a pathology or a state of malaise due to vitamin B12 deficiencies or deficits.

In another preferred embodiment, said pharmaceutical composition is indicated for use in the preventive and/or curative treatment of subjects suffering from pernicious anaemia.

In another preferred embodiment, said pharmaceutical composition is indicated for use in the treatment of subjects who take vitamin C.

In another preferred embodiment, said composition or supplement can comprise said vitamin B12 producing bacterial strains and/or said bacterial strains capable of accumulating selenium and/or said folic acid producing bacterial strains coated with a composition containing at least one lipid, preferably of vegetable origin, said lipid being selected from the group comprising saturated fats having a melting point below 75°C, preferably comprised from 45 to 65°C.

### Experimental part - Microbiological method

The present microbiological method is used to determine the amount of vitamin B12 produced by the bacterial cells of the strains of the present invention.

The method is based on the use of the strain *Lactobacillus delbrueckii subs. lactis (L. leichmannii)* ATCC 7830-DSMZ 20355, which is auxotrophic for vitamin B12 and thus grows proportionally to the amount of vitamin B12 present in the culture medium. This growth is determined by spectrophotometric reading of the culture at a wavelength of 600 nm. The calibration line calculated from the growth of *Lactobacillus delbrueckii subs. lactis (L. leichmannii)* in media containing known scalar quantities of vitamin B12 is then used to determine the cobalamin produced by the bacterial strains of the present invention which were submitted to analysis..

### Microbiological method

### 1.1 Preparation of the auxotrophic strain for calculating the calibration line and concentrations of vitamin B12 produced.

1.1.1 The auxotrophic strain *Lactobacillus delbrueckii subs. lactis (L. leichmannii)* ATCC 7830-DSMZ 20355 is inoculated at a percentage of 1% in 15 ml of MRS broth (MRS Difco 55.00 g and 1000 ml of distilled water) and 1% cysteine hydrochloride is added to it (Merck code 1.02735.0100 - 50.00 g and 1000 ml of distilled water; 5% sol.).
1.1.2 It is subsequently vortexed in a vortex mixer.
1.1.3 The screw cap is screwed down taking care not to tighten it completely in order to enable the development of an anoxic environment. The strain is incubated in a Gas-Pack provided with an Anaerocult A anaerobic system for 18-24 hours at 37 °C.
1.1.4 Subsequently, the culture obtained is transplanted two consecutive times using the same medium and the same percentage of inoculum.
1.1.5 At the end of the incubation period, centrifuge the culture of *Lactobacillus delbrueckii subs. lactis (L. leichmannii)* ATCC 7830-DSMZ 20355 at 6000 rpm for 15 minutes.
11.1.6 At the end of centrifugation, the supernatant is discarded and the pellet is washed three consecutive times with 10 ml of 0.85% NaCl saline solution.
1.1.7 The pellet is resuspended in 10 ml of 0.85% NaCl saline solution.
1.1.8 Then a 1:100 dilution is made with 0.85% NaCl saline solution so as to obtain the inoculum necessary for setting up tubes at known concentrations of vitamin B12, necessary for calculating the calibration line and setting up tubes from which the amount of vitamin B12 produced will be determined.

### 1.2 Preparation of the tubes necessary for calculating the calibration line.

1.2.1 7.6 g of vitamin B12 Assay Medium (Difco code 60179) is dissolved in 100 ml of ultrapure water.
1.2.2 It is heated and stirred simultaneously and allowed to boil for about 2-3 minutes to completely dissolve all the powder.
1.2.3 5 ml of vitamin B12 Assay Medium is distributed into seven glass test tubes previously washed with ultrapure water and placed in an oven at 100°C overnight. 1.2.4 The stock solution of cyanocobalamin (prepared as described below) is added to the test tubes, which are brought to the final volume of 10 ml with ultrapure water, as described below:
   - ml of stock solution per tube:
      0, 0.5, 1, 2, 3, 4, 5
   - ml of ultrapure water per tube:
      5, 4.5, 4, 3, 2, 1, 0
   - Concentration of vitamin B12 per tube (ng/ml):
      0.0 0.025 0.05 0.1 0 0.15 0.20 0.25

   The stock solution of cyanocobalamin is prepared as follows:
   i) Dissolve 10 mg of cyanocobalamin standard (Sigma code C3607) in a 100 ml flask, bringing it to the required volume with a 25% ethanol solution in ultrapure water (final concentration 0.1 mg/ml).
   ii) Add 1 ml of the solution obtained in step (i) to 99 ml of ultrapure water (final concentration 1 µg/ml).
   iii) Add 1 ml of the solution obtained in step (ii) to 99 ml of ultrapure water (final concentration 10 ng/ml).
   iv) Add 1 ml of the solution obtained in step (iii) to 199 ml of ultrapure water (final concentration 0.05 ng/ml). 1.2.5 The tubes are autoclaved at 121°C for 15 minutes.
1.2.6 At the end of the heat treatment, add 100 µl of the suspension of *Lactobacillus delbrueckii subsp. lactis (L. leichmannii)* ATCC 7830-DSMZ 20355, obtained as described above in 1.1.8, to each test tube.
1.2.7 The tubes are subsequently vortexed in a vortex mixer.
1.2.8 The screw cap is screwed down taking care not to tighten it completely in order to enable the development of an anoxic environment. The strain is incubated in a Gas-Pack provided with an Anaerocult A anaerobic system for 18-24 hours at 37°C.
1.2.9 At the end of the incubation period, chill the test tubes at 4°C for 15-20 minutes to block bacterial growth.

### 1.3 Calculation of the calibration line.

1.3.1 The spectrophotometer is reset to 660 nm against 1 ml of vitamin B12 Assay medium (dilution with 0 ng/ml of cyanocobalamin) obtained as described above.
11.3.2 Add 1 ml of the culture obtained as per step 1.2.9 in 1.5 ml plastic cuvettes according to the ascending order of the concentration of cyanocobalamin present.
1.3.3 The absorbance values are read at the wavelength of 660 nm.
1.3.4 The absorbance values obtained were used to determine the calibration line, whose equation will be used to extrapolate data related to the unknown quantities of vitamin B12 present in the cultures obtained as per step 1.4.18.

### 1.4 Preparation of samples for determining the vitamin B12 produced.

1.4.1 7.6 g of Vitamin B12 Assay Medium is dissolved in 100 ml of ultrapure water.
1.4.2 It is heated and stirred simultaneously and allowed to boil for about 2-3 minutes to completely dissolve all the powder.
1.4.3 5 ml of vitamin B12 Assay Medium is distributed into "n" glass test tubes (n = number of samples to be activated x number of activation subcultures) previously washed with ultrapure water and placed in an oven at 100°C.
1.4.4 5 ml of vitamin B12 Assay Medium is distributed into "n" glass test tubes (n = number of samples to be analyzed x 5) previously washed with ultrapure water and placed in an oven at 100°C overnight.
1.4.5 25 ml of vitamin B12 Assay medium is distributed into "n" 100 ml Bibby beakers (Pyrex glass bottles, generally 100 or 250 ml, suitable for autoclave sterilization of culture media) (n = number of samples to be analyzed) previously washed with ultrapure water and placed in an oven at 100°C.
1.4.6 The test tubes as described in step 1.4.3 are brought to a volume of 10 ml with the addition of ultrapure water.
1.4.7 The Bibby beakers as per step 1.4.5 are brought to a volume of 50 ml with the addition of ultrapure water.
1.4.8 They are autoclaved at 121°C for 15 minutes.
1.4.9 The bacterial strains to be analyzed are inoculated in the test tubes belonging to the group described in step 1.4.3, at a percentage of 1%.
1.4.10 The tubes are subsequently vortexed in a vortex mixer.
1.4.11 The screw cap is firmly screwed down and the tubes are incubated in a temperature-controlled bath for 18-24 hours at 37°C.
1.4.12 The culture obtained is transplanted three consecutive times using the same medium and the same percentage of inoculum.
1.4.13 The bacterial strains in the Bibby beakers belonging to the group described in step 1.4.5 are inoculated, at a percentage of 1%.
1.4.14 The beakers are subsequently vortexed in a vortex mixer.
1.4.15 The screw cap is firmly screwed down and the cultures are incubated in a temperature-controlled bath for 18-24 hours at 37°C.
1.4.16 At the end of the incubation period, centrifuge at 6000 rpm for 15 minutes.
1.4.17 At the end of centrifugation, the supernatant is removed and the pellet is washed three consecutive times with 10 ml of 0.1 M pH 7 sodium phosphate buffer.
1.4.18 The pellet is resuspended in 1 ml of 0.1 M pH 4.5 sodium phosphate extraction buffer [Citric acid], 0.005% KCN.
   The extraction buffer is prepared as follows:
   a1) Bring 100 ml of 0.1 M pH 7 sodium phosphate buffer solution to a pH of 4.5 by adding citric acid.
   a2) Add 0.005% KCN to the solution obtained in step a1).
   a3) Submit to stirring for 10 minutes.
1.4.19 The solution is submitted to stirring at 4°C with 1 g of glass beads for three 5-minute cycles.
1.4.20 At the end of the stirring period, the extract is boiled at 100°C for 5 minutes to permit protein denaturation and formation of cyanocobalamin.
1.4.21 At the end of the heat treatment, the extract is diluted with ultrapure water according to the proportions shown below:
   - dilution: 1:2 1:5 1:10 1:100 1:1000
   - µl extracted: 500 200 100 10 1
   - µl ddH₂O: 500 800 900 990 999
1.4.22 Each dilution is added to the tubes as per step 1.4.4.
1.4.23 Each tube is brought to a volume of 10 ml with ultrapure water.
1.4.24 100 µl of the suspension of *Lactobacillus delbrueckii subs. lactis (L*. *leichmannii)* ATCC 7830-DSMZ 20355 as per step 1.4.8 is added.
1.4.25 The tubes are subsequently vortexed in a vortex mixer.
1.4.26 The screw cap is screwed down taking care not to tighten it completely in order to enable the development of an anoxic environment. The strain is incubated in a Gas-Pack provided with an Anaerocult A anaerobic system for 18-24 hours at 37°C.
1.4.27 At the end of the incubation period, the test tubes are chilled at 4°C for 15-20 minutes to block bacterial growth.

### 1.5 Determination of the vitamin B12 produced.

1.5.1 The spectrophotometer is reset to 660 nm against 1 ml of vitamin B12 Assay medium (dilution with 0 ng/ml of cyanocobalamin) obtained as described above.
1.5.2 1 ml of the culture obtained as described in step 1.2.9 is added to 1.5 ml plastic cuvettes according to the ascending order of the dilutions of the extract as described in step 1.4.21.
1.5.3 The absorbance values are read at the wavelength of 660 nm.
1.5.4 The absorbance values obtained were used in the equation of the line calculated above in step 1.3.4 to calculate the concentration of vitamin B12 produced by the strains considered. The results are expressed in ng/l.

### Experimental part - Analytical method (HPLC)

The present analytical method is used to determine the amount of vitamin B12 produced by the bacterial cells of the strains of the present invention.

The analytical determination of the vitamin B12 produced by the bacterial cultures of the strains of the present invention, which are the subject of the present analysis, is performed with the reverse phase HPLC method (column C18) using a UV/VIS detector.

### Analytical method (HPLC)

### 2.1 Preparation of standard solutions for calculating the calibration line.

2.1.1 10 mg of Cyanocobalamin Standard (Sigma code C3607) is weighed and the following serial dilutions are made:
   2.1.1.1 1 mg/ml solution: weigh 10 mg of Cyanocobalamin Standard (Sigma code C3607) in a 10 ml flask and bring to the required volume with Milli-Q water and 25% ethanol (ethanol serves to increase the solubility of the cyanocobalamin).
   2.1.1.2 0.1 mg/ml solution: take 1 ml of the solution as per step 2.2.1.1 and bring it to the required volume in a 10 ml flask with Milli-Q water.
   2.1.1.3 0.01 mg/ml solution: take 1 ml of the solution as per step 2.2.1.2 and bring it to the required volume in a 10 ml flask with Milli-Q water.
   2.1.1.4 Stock solution (1000 ng/ml): take 5 ml of the solution as per step 2.2.1.3 and bring it to the required volume in a 50 ml flask with Milli-Q water.
2.1.2 The stock solution described in step 2.2.1.4 is used to prepare standard dilutions for creating the calibration line in the following manner:
   2.1.2.1 1000 ng/ml solution or stock solution as such.
   2.1.2.2 800 ng/ml solution: take 4 ml of stock solution and bring to the required volume in a 5 ml flask with Milli-Q water.
   2.1.2.3 500 ng/ml solution: take 5 ml of stock solution and bring to the required volume in a 10 ml flask with Milli-Q water.
   2.1.2.4 200 ng/ml solution: take 1 ml of stock solution and bring to the required volume in a 5 ml flask with Milli-Q water.
   2.1.2.5 100 ng/ml solution: take 1 ml of stock solution and bring to the required volume in a 10 ml flask with Milli-Q water.
   2.1.2.6 50 ng/ml solution: take 1 ml of the 500 ng/ml solution (see step 2.2.23) and bring to the required volume in a 10 ml flask with Milli-Q water.
   2.1.2.7 25 ng/ml solution: take 5 ml of the 50 ng/ml solution (see step 7.2.2.6) and bring to the required volume in a 10 ml flask with Milli-Q water.

### 2.2 Main parameter settings for the HPLC method.

In order to use the HPLC Waters 625 LC System, TotalChrom (Perkin Elmer) data acquisition software and the UV/VIS 785 A detector (Perkin Elmer) correctly, the directions provided in the usage procedures must be followed carefully. The fundamental test parameters for determining the amount of Vitamin B12 produced by the bacterial strains submitted to analysis are indicated below:
2.2.1 HPLC parameters:
- Type of chromatography column:
Spherisorb ODS 2 column (Waters) C18 250x4.6 mm, diameter 5 µm.

- Mobile phase: Milli-Q water and Acetonitrile in elution gradient (as indicated in the table below).
- Elution gradient: set the elution gradient from the keyboard of the HPLC Waters 625 LC System controller, as shown below:

| Time | % Milli-Q Water | % Acetonitrile |
|---|---|---|
| 0 | 95 | 5 |
| 14 | 85 | 15 |
| 19 | 70 | 30 |
| 20 | 95 | 5 |
| 35 | 95 | 5 |

- Elution flow: On the Waters 625 LC HPLC System controller, set a constant flow of 1.0 ml/min.
- Column temperature: On the Waters 625 LC HPLC System controller set a temperature of 25°C for the temperature-controlled oven of the column.
- Injection volume: 20 µl of standard and sample.
2.2.2 Parameters for the UV/VIS detector. The wavelength is equal to 360 nm.

### 2.3 Calibration line.

2.3.1 Inject all the standard solutions prepared as described in step 2.2, one after the other.
2.3.2 Acquire the associated chromatograms via the TotalChrom software.
2.3.3 The software automatically integrates the peaks present in each chromatogram. However, it is fundamental to check that the automatic integration performed by the software is correct. The retention time of cyanocobalamin is about 19 minutes.
2.3.4 The areas of each single peak associated with vitamin B12 are recorded. Based on the value of the areas of the peaks in relation to the starting concentrations, a scatter plot is created.
2.3.5 The calibration line generated must have a value of R2 > 0.99.
2.3.6 The vitamin B12 present in the samples submitted to analysis is calculated by interpolation from the calibration line.

### 2.4 Preparation of samples for determining the vitamin B12 produced.

The samples are prepared following the same method as described in steps 1.4.1 to 1.4.20.

### 2.5 Determination of the vitamin B12 produced.

2.5.1 20 µl of the extract prepared in step 1.4.20 is injected in HPLC.
2.5.2 The value of the area of the peak associated with the sample analyzed is interpolated from the calibration line prepared as per step 1.4.
2.5.3 Calculate the final vitamin B12 value of each individual sample based on the quantity of the injected sample (20 µl). The results are expressed in ng/l.

The Applicant carried out an HPLC assay (mean of a number of tests) [vitamin B12 concentration expressed in µg/litre] at OD₆₀₀ and determined the following values:
- *Lactobacillus reuteri* DSM 23878 (LRE02) = 45.57
- *Lactobacillus reuteri* ATCC 55730 = 43.02
- *Lactobacillus reuteri* DSM 17938 = 32.27

**Table 1**

| **VITAMIN B12 producing strains** | **Microbiological assay** (mean of a number of tests) [vitamin B12 concentration expressed in µg/litre] at OD₆₀₀ | **HPLC assay** (mean of a number of tests) [vitamin B12 concentration expressed in µg/litre] at OD₆₀₀ |
|---|---|---|
| *Lactobacillus reuteri* **ATCC 55730** | 15,640 ± 75 % | 18,030 ± 12% |
| *Lactobacillus reuteri* **DSM 17938** | 10,950 ± 68 % | 12,890 ± 10% |
| *Lactobacillus reuteri* **DSM 16143** | 2,790 ± 76 % | 8,000 ± 13% |

## Claims

1. A composition comprising probiotic bacterial strain belonging to the species *Lactobacillus reuteri,* as a vitamin B12 producer, identified as *Lactobacillus reuteri* DSM 23878 - (LRE02) and at least one strain selected from the group consisting of:
- *Lactobacillus reuteri* ATCC 55730,
- *Lactobacillus reuteri* DSM 17938, and
- *Lactobacillus reuteri* DSM 16143;
more preferably comprising the strain *Lactobacillus reuteri* DSM 23878 (LRE02) and at least one strain selected from the group consisting of:
- *Lactobacillus reuteri* ATCC 55730,
- *Lactobacillus reuteri* DSM 17938.

2. A food or nutraceutical composition or supplement or pharmaceutical composition comprising at least one bacterial strain belonging to the species *Lactobacillus reuteri,* as a vitamin B12 producer, identified as *Lactobacillus reuteri* DSM 23878 - (LRE02), for use in the preventive and/or curative treatment of subjects suffering from a pathology due to vitamin B12 deficiencies.

3. The food or nutraceutical composition or supplement or pharmaceutical composition for use according to claim 2, wherein said composition comprises:
i) the strain *Lactobacillus reuteri* ATCC 55730 and *Lactobacillus reuteri* DSM 23878 (LRE02), or
ii) the strain *Lactobacillus reuteri* DSM 17938 and *Lactobacillus reuteri* DSM 23878 (LRE02), or
iii) the strain *Lactobacillus reuteri* DSM 16143 and *Lactobacillus reuteri* DSM 23878 (LRE02), or
iv) the three strains *Lactobacillus reuteri* ATCC 55730, *Lactobacillus reuteri* DSM 17938 and *Lactobacillus reuteri* DSM 23878 (LRE02), or
v) the three strains *Lactobacillus reuteri* DSM 23878 (LRE02), *Lactobacillus reuteri* DSM 17938 and *Lactobacillus reuteri* DSM 16143, or
vi) the three strains *Lactobacillus reuteri* DSM 23878 (LRE02), *Lactobacillus reuteri* ATCC 55730 and *Lactobacillus reuteri* DSM 16143.

4. The food or nutraceutical composition or supplement or pharmaceutical composition for use according to claim 3, wherein at least one further bacterial strain capable of accumulating selenium inside the bacterial cells is additionally present; said bacterial strain is preferably selected from the group consisting of:
*Lactobacillus buchneri* LB26BM, deposited with the DSMZ on 05/04/2004 and having the deposit number DSM 16341, *Lactobacillus ferintoshensis* LB6BM, deposited with the DSMZ on 17/01/2004 and having the deposit number DSM 16144, and *Lactobacillus reuteri* LB2BM, deposited with the DSMZ on 17/01/2004 and having the deposit number DSM 16143.

5. The food or nutraceutical composition or supplement or pharmaceutical composition for use according to claim 3 or 4, wherein glutathione in reduced form is additionally present.

6. The food or nutraceutical composition or supplement or pharmaceutical composition for use according to any one of claims 3-5, wherein a further bacterial strain capable of producing folic acid is additionally present, preferably said strain is selected from the group consisting of: a bacterial strain belonging to the species *Bifidobacterium adolescentis,* preferably *Bifidobacterium adolescentis* deposited with the DSMZ on 21.07.2004 and having the accession number DSM 16595; a bacterial strain belonging to the species *Bifidobacterium catenulatum*/*pseudocatenulatum,* preferably *Bifidobacterium catenulatum*/*pseudocatenulatum* deposited with the DSMZ on 15.06.2006 and having the accession number DSM 18350; a bacterial strain belonging to the species *Bifidobacterium animalis subsp. lactis,* preferably *Bifidobacterium animalis subsp. lactis* deposited with the DSMZ on 15.06.2006 and having the accession number DSM 18352; a bacterial strain belonging to the species *Bifidobacterium breve,* preferably *Bifidobacterium breve* deposited with the DSMZ on 21.07.2004 and having the accession number DSM 16596; a bacterial strain belonging to the species *Bifidobacterium pseudocatenulatum,* preferably *Bifidobacterium pseudocatenulatum* deposited with the DSMZ on 21.07.2004 and having the accession number DSM 16597, *Bifidobacterium pseudocatenulatum* deposited with the DSMZ on 21.07.2004 and having the accession number DSM 16598 and *Bifidobacterium catenulatum*/*pseudocatenulatum* deposited with the DSMZ on 15.06.2006 and having the accession number DSM 18353.

7. The pharmaceutical composition according to any one of claims 3-6, for use in the preventive and/or curative treatment of subjects suffering from pernicious anaemia, or for use in the preventive and/or curative treatment of cobalamin deficiencies in subjects who take vitamin C.

8. The composition for use according to any one of claims 3 to 7, wherein said vitamin B12 producing bacterial strains and/or said bacterial strains capable of accumulating selenium and/or said folic acid producing bacterial strains are coated with a composition containing at least one lipid, preferably of vegetable origin, said lipid being selected from the group comprising saturated fats having a melting point below 75°C, preferably comprised from 45 to 65°C.

## Patentansprüche

1. Zusammensetzung, umfassend einen probiotischen Bakterienstamm, der zu der Spezies *Lactobacillus reuteri* gehört, als Vitamin B12 Produzent, identifiziert als *Lactobacillus reuteri* DSM 23878 (LRE02), und wenigstens einen Stamm, ausgewählt aus der Gruppe bestehend aus:
- *Lactobacillus reuteri* ATCC 55730,
- *Lactobacillus reuteri* DSM 17938 und
- *Lactobacillus reuteri* DSM 16143;
vorzugsweise umfassend den Stamm *Lactobacillus reuteri* DSM 23878 (LRE02) und wenigstens einen Stamm, ausgewählt aus der Gruppe bestehend aus:
- *Lactobacillus reuteri* ATCC 55730,
- *Lactobacillus reuteri* DSM 17938.

2. Lebensmittel oder nutrazeutische Zusammensetzung oder Ergänzungsmittel oder pharmazeutische Zusammensetzung, umfassend wenigstens einen Bakterienstamm der Spezies *Lactobacillus reuteri* als Vitamin B12-Produzenten, identifiziert als *Lactobacillus reuteri* DSM 23878 - (LRE02), zur Verwendung bei der präventiven und/oder kurativen Behandlung von Patienten, die an einer Pathologie aufgrund von Vitamin B12-Mangel leiden.

3. Nahrungsmittel oder nutrazeutische Zusammensetzung oder Ergänzungsmittel oder pharmazeutische Zusammensetzung zur Verwendung gemäß Anspruch 2, wobei die Zusammensetzung umfasst:
i) den Stamm *Lactobacillus reuteri* ATCC 55730 und *Lactobacillus reuteri* DSM 23878 (LRE02), oder
ii) den Stamm *Lactobacillus reuteri* DSM 17938 und *Lactobacillus reuteri* DSM 23878 (LRE02), oder
iii) den Stamm *Lactobacillus reuteri* DSM 16143 und *Lactobacillus reuteri* DSM 23878 (LRE02), oder
iv) die drei Stämme *Lactobacillus reuteri* ATCC 55730, *Lactobacillus reuteri* DSM 17938 und *Lactobacillus reuteri* DSM 23878 (LRE02), oder
v) die drei Stämme *Lactobacillus reuteri* DSM 23878 (LRE02), *Lactobacillus reuteri* DSM 17938 und *Lactobacillus reuteri* DSM 16143, oder
vi) die drei Stämme *Lactobacillus reuteri* DSM 23878 (LRE02), *Lactobacillus reuteri* ATCC 55730 und *Lactobacillus reuteri* DSM 16143.

4. Nahrungsmittel oder nutrazeutische Zusammensetzung oder Ergänzungsmittel oder pharmazeutische Zusammensetzung zur Verwendung gemäß Anspruch 3, wobei außerdem wenigstens ein weiterer Bakterienstamm, der in der Lage ist, in den bakteriellen Zellen Selen zu akkumulieren, vorhanden ist; wobei dieser Bakterienstamm vorzugsweise ausgewählt wird aus der Gruppe bestehend aus:
*Lactobacillus buchneri* LB26BM, hinterlegt beim DSMZ am 05.04.2004 mit der Hinterlegungsnummer DSM 16341, *Lactobacillus ferintoshensis* LB6BM, hinterlegt beim DSMZ am 17.01.2004 mit der Hinterlegungsnummer DSM 16144, und *Lactobacillus reuteri* LB2BM, hinterlegt beim DSMZ am 17.01.2004 mit der Hinterlegungsnummer DSM 16143.

5. Nahrungsmittel oder nutrazeutische Zusammensetzung oder Ergänzungsmittel oder pharmazeutische Zusammensetzung zur Verwendung gemäß Anspruch 3 oder 4, wobei außerdem Glutathion in reduzierter Form vorhanden ist.

6. Nahrungsmittel oder nutrazeutische Zusammensetzung oder Ergänzungsmittel oder pharmazeutische Zusammensetzung zur Verwendung gemäß einem der Ansprüche 3 bis 5, in der außerdem ein weiterer Bakterienstamm vorliegt, der in der Lage ist, Folsäure zu produzieren, wobei dieser Stamm vorzugsweise ausgewählt wird aus der Gruppe bestehend aus: einem Bakterienstamm der Spezies *Bifidobacterium adolescentis,* vorzugsweise *Bifidobacterium adolescentis,* hinterlegt beim DSMZ am 21.07.2004 mit der Hinterlegungsnummer DSM 16595; einem Bakterienstamm der Spezies *Bifidobacterium catenulatum*/*pseudocatenulatum,* vorzugsweise *Bifidobacterium catenulatum*/*pseudocatenulatum,* hinterlegt beim DSMZ am 15.06.2006 mit der Hinterlegungsnummer DSM 18350; einem Bakterienstamm der Spezies *Bifidobacterium animalis subsp. lactis,* vorzugsweise *Bifidobacterium animalis subsp. Lactis,* der beim DSMZ am 15.06.2006 hinterlegt wurde und die Zugangsnummer DSM 18352 hat; einem Bakterienstamm der Spezies *Bifidobacterium breve,* vorzugsweise *Bifidobacterium breve,* hinterlegt beim DSMZ am 21.07.2004 mit der Zugangsnummer DSM 16596; einem Bakterienstamm der Spezies *Bifidobacterium pseudocatenulatum,* vorzugsweise *Bifidobacterium pseudocatenulatum,* hinterlegt beim DSMZ am 21.07.2004 mit der Zugangsnummer DSM 16597, *Bifidobacterium pseudocatenulatum,* hinterlegt beim DSMZ am 21.07.2004 mit der Zugangsnummer DSM 16598, und *Bifidobacterium catenulatum*/*pseudocatenulatum,* hinterlegt beim DSMZ am 15.06.2006 mit der Zugangsnummer DSM 18353.

7. Pharmazeutische Zusammensetzung gemäß einem der Ansprüche 3 bis 6 zur Verwendung bei der präventiven und/oder kurativen Behandlung von Patienten, die an perniziöser Anämie leiden, oder zur Verwendung bei der präventiven und/oder kurativen Behandlung von Cobalaminmängeln bei Patienten, die Vitamin C nehmen.

8. Zusammensetzung zur Verwendung gemäß einem der Ansprüche 3 bis 7, wobei die Vitamin B12-produzierenden Bakterienstämme und/oder die Bakterienstämme, die zur Akkumulation von Selen in der Lage sind, und/oder die Folsäure-produzierenden Bakterienstämme mit einer Zusammensetzung beschichtet sind, die wenigstens ein Lipid, vorzugsweise pflanzlichen Ursprungs, enthält, wobei dieses Lipid aus der Gruppe bestehend aus gesättigten Fetten mit einem Schmelzpunkt unter 75°C, vorzugsweise zwischen 45 bis 65°C, ausgewählt wird.

## Revendications

1. Composition comprenant une souche bactérienne probiotique appartenant aux espèces *Lactobacillus reuteri,* à titre de souche productrice de vitamine B12, identifiée comme étant la souche *Lactobacillus reuteri* DSM 23878 - (LRE02) et au moins une souche choisie dans le groupe constitué par :
- *Lactobacillus reuteri* ATCC 55730,
- *Lactobacillus reuteri* DSM 17938, et
- *Lactobacillus reuteri* DSM 16143 ;
plus préférablement comprenant la souche *Lactobacillus reuteri* DSM 23878 (LRE02) et au moins une souche choisie dans le groupe constitué par :
- *Lactobacillus reuteri* ATCC 55730,
- *Lactobacillus reuteri* DSM 17938.

2. Aliment ou composition nutraceutique ou complément alimentaire ou composition pharmaceutique comprenant au moins une souche bactérienne appartenant aux espèces *Lactobacillus reuteri,* à titre de souche productrice de vitamine B12, identifiée comme étant la souche *Lactobacillus reuteri* DSM 23878 - (LRE02), pour son utilisation dans le traitement préventif et/ou curatif des sujets souffrant d'une pathologie imputable à des carences en vitamine B12.

3. Aliment ou composition nutraceutique ou complément alimentaire ou composition pharmaceutique pour son utilisation selon la revendication 2, dans lequel ladite composition comprend :
i) la souche *Lactobacillus reuteri* ATCC 55730 et *Lactobacillus reuteri* DSM 23878 (LRE02), ou
ii) la souche *Lactobacillus reuteri* DSM 17938 et *Lactobacillus reuteri* DSM 23878 (LRE02), ou
iii) la souche *Lactobacillus reuteri* DSM 16143 et *Lactobacillus reuteri* DSM 23878 (LRE02), ou
iv) les trois souches *Lactobacillus reuteri* ATCC 55730, *Lactobacillus reuteri* DSM 17938 et *Lactobacillus reuteri* DSM 23878 (LRE02), ou
v) les trois souches *Lactobacillus reuteri* DSM 23878 (LRE02), *Lactobacillus reuteri* DSM 17938 et *Lactobacillus reuteri* DSM 16143, ou
vi) les trois souches *Lactobacillus reuteri* DSM 23878 (LRE02), *Lactobacillus reuteri* ATCC 55730 et *Lactobacillus reuteri* DSM 16143.

4. Aliment ou composition nutraceutique ou complément alimentaire ou composition pharmaceutique pour son utilisation selon la revendication 3, dans lequel au moins une autre souche bactérienne capable d'accumuler du sélénium à l'intérieur des cellules bactériennes est en plus présente ; ladite souche bactérienne est de préférence choisie dans le groupe constitué par :
la souche *Lactobacillus buchneri* LB26BM, déposée auprès de la Collection DSMZ le 05/04/2004 et portant le numéro de dépôt DSM 16341, la souche *Lactobacillus ferintoshensis* LB6BM, déposée auprès de la Collection DSMZ le 17/01/2004 et portant le numéro de dépôt DSM 16144, et la souche *Lactobacillus reuteri* LB2BM, déposée auprès de la Collection DSMZ le 17/01/2004 et portant le numéro de dépôt DSM 16143.

5. Aliment ou composition nutraceutique ou complément alimentaire ou composition pharmaceutique pour son utilisation selon la revendication 3 ou 4, dans lequel du glutathion sous forme réduite est en plus présent.

6. Aliment ou composition nutraceutique ou complément ou composition pharmaceutique pour son utilisation selon l'une quelconque des revendications 3 à 5, dans lequel une autre souche bactérienne capable de produire de l'acide folique est en plus présente, de préférence ladite souche est choisie dans le groupe constitué par : une souche bactérienne appartenant aux espèces *Bifidobacterium adolescentis,* de préférence la souche *Bifidobacterium adolescentis* déposée auprès de la Collection DSMZ le 21.07.2004 et portant le numéro d'accès DSM 16595 ;
une souche bactérienne appartenant aux espèces *Bifidobacterium catenulatum*/*pseudocatenulatum,* de préférence la souche *Bifidobacterium catenulatum*/*pseudocatenulatum* déposée auprès de la Collection DSMZ le 15.06.2006 et portant le numéro d'accès DSM 18350 ; une souche bactérienne appartenant aux espèces *Bifidobacterium animalis ssp. lactis,* de préférence la souche *Bifidobacterium animalis ssp. lactis* déposée auprès de la Collection DSMZ le 15. 06.2006 et portant le numéro d'accès DSM 18352 ; une souche bactérienne appartenant aux espèces *Bifidobacterium breve,* de préférence la souche *Bifidobacterium breve* déposée auprès de la Collection DSMZ le 21.07.2004 et portant le numéro d'accès DSM 16596 ; une souche bactérienne appartenant aux espèces *Bifidobacterium pseudocatenulatum,* de préférence la souche *Bifidobacterium pseudocatenulatum* déposée auprès de la Collection DSMZ le 21.07.2004 et portant le numéro d'accès DSM 16597, la souche *Bifidobacterium pseudocatenulatum* déposée auprès de la Collection DSMZ le 21.07.2004 et portant le numéro d'accès DSM 16598 et la souche *Bifidobacterium catenulatum*/*pseudocatenulatum* déposée auprès de la Collection DSMZ le 15.06.2006 et portant le numéro d'accès DSM 18353.

7. Composition pharmaceutique selon l'une quelconque des revendications 3 à 6, pour son utilisation dans le traitement préventif et/ou curatif des sujets souffrant d'anémie pernicieuse, ou pour son utilisation dans le traitement préventif et/ou curatif des carences en cobalamine chez les sujets sous vitamine C.

8. Composition pour son utilisation selon l'une quelconque des revendications 3 à 7, dans laquelle lesdites souches bactériennes productrices de vitamine B12 et/ou lesdites souches bactériennes capables d'accumuler du sélénium et/ou lesdites souches bactériennes productrices d'acide folique sont revêtues d'une composition contenant au moins un lipide, de préférence d'origine végétale, ledit lipide étant choisi dans le groupe comprenant les graisses saturées ayant un point de fusion inférieur à 75 °C, de préférence compris entre 45 et 65 °C.
